# EUROPEAN PATENT APPLICATION

(11) **EP 2 218 770 A1**
(43) Date of publication of application: **18.08.2010**
(21) Application number: 08859611.9
(22) Date of filing: 05.12.2008
(51) Int. Cl.: C12M 1/00, C12N 15/09

(54) **CHIP DRIVE**

(30) Priority: 10.12.2007 JP 2007318888
(71) Applicant: Olympus Corporation, Shibuya-ku Tokyo 151-0072 (JP)
(72) Inventor: IMAOKA, Yuka, Tokyo 192-8512 (JP); SASAKI, Yasuo, Tokyo 192-8512 (JP); TATEYAMA, Kiyohiko, Tokyo 192-8512 (JP)
(74) Representative: Winter, Brandl, Fürniss, Hübner, Röss, Kaiser, Polte - Partnerschaft
(86) International application number: PCT/JP2008/072193
(87) International publication number: WO 2009/075234

(57) **Abstract**

A tip drive apparatus (10) capable to moving a tip unit (42) toward a cell in a dish (22), while holding the tip unit at a prescribed angle, the tip unit being formed on a lever unit (60) having flexibility and directed to the cell at the prescribed angle, includes a needle (44) having a shaft (56) to which the support unit on which the tip unit is formed, and an adaptor (46) for holding the needle (44), enabling the needle to be replaced by another, the adaptor being configured to be able to change a positional relation with respect to the main unit (30) of the tip drive apparatus, while the needle (44) is being attached.

## Description

### Technical Field

The present invention relates to a tip drive apparatus that can move a tip unit formed on a support unit having flexibility and arranged at a prescribed angle to an object, while holding the tip unit at the prescribed angle.

### Background Art

WO 04/092369 discloses a microinjection method and apparatus that are designed to introduce a substance, such as genes, into cell. In the technique disclosed, the substance is electrically adsorbed to the distal end of a microneedle, which is a tip unit, and the microneedle is then inserted into a cell. A pulse voltage is applied to the microneedle, moving the substance from the distal end of the microneedle and introducing the substance into the cell. The microneedle is thrust into the cell as it is minutely moved by using a piezoelectric element that can expand and contract coaxially with the microneedle. This technique is to hold genes at the distal end of the microneedle, can introduce the genes into the cell in a low-invasive manner, increasing the survival rate of the cell.

However, the microneedle cannot penetrate the cell membrane in some cases. This is because the microneedle invades the cell at a very small volume and also because the cell membrane has fluidity. Even if the microneedle is moved to such a position where its distal end may penetrate the cell membrane, the cell membrane covers up the surface of the distal end, disabling the needle from piercing the cell membrane in some cases. Consequently, the tip cannot be stably driven and the substance cannot be introduced at a sufficient rate.

The technique disclosed in the above-identified document cannot utilize a tip drive apparatus that supplies an electric current to the microneedle in order to give an electrical stimulus to a living cell so that the cell may be observed in a living state with high efficiency.

### Disclosure of Invention

This invention has been made in view of the foregoing. An object of the invention is to provide an apparatus for driving a tip, which can introduce a substance into a cell at in a low-invasive manner, thus maintaining the cell at a high survival rate or apply an electrical stimulus to the cell in order to observe the living cell with high efficiency.

According to an aspect of embodiments, there is provided a tip drive apparatus capable to moving a tip unit toward an object, while holding the tip unit at a prescribed angle, the tip unit being formed on a support unit having flexibility and directed to the object at the prescribed angle, the apparatus comprising:
a needle having a shaft to which the support unit on which the tip unit is formed; and
an adaptor for holding the needle, enabling the needle to be replaced by another,
the adaptor being configured to be able to change a positional relation with respect to the main unit of the tip drive apparatus, while the needle is being attached.

### Brief Description of Drawings

FIG. 1 is a diagram showing the overall configuration of a tip drive apparatus according to a first embodiment of this invention.
FIG. 2 is a diagram showing the characterizing section of the tip drive apparatus according to the first embodiment.
FIG. 3 is a diagram showing the configuration of a needle.
FIG. 4 is a diagram explaining the interference that may be caused, depending on the angle of the needle.
FIG. 5 is a diagram explaining the region in which the needle can move.
FIG. 6 is a block diagram showing the electrical configuration of the tip drive apparatus according to the first embodiment.
FIG. 7 is a flowchart explaining a tip driving method using the tip drive apparatus according to the first embodiment.
FIG. 8 is a diagram presenting a microscope image of HelaS3 cells into which tip drive apparatus according to the first embodiment has introduced genes to express GFP fluorescent protein.
FIG. 9 is a diagram presenting an image of the HelaS3 cells, acquired through a phase contrast microscope, 24 hours after the genes had been introduced into the cell to express GFP fluorescent protein.
FIG. 10 is a diagram presenting a microscope image of the HelaS3 cells, acquired through a fluorescence observation, 24 hours after the genes had been introduced into the cell to express GFP fluorescent protein.
FIG. 11 is a diagram showing the configuration of the characterizing section of a tip drive apparatus according to a second embodiment of this invention.
FIG. 12 is a diagram showing the configuration of the characterizing section of a tip drive apparatus according to a third embodiment of this invention.
FIG. 13 is a diagram showing the configuration of the characterizing section of a tip drive apparatus according to a fourth embodiment of the present invention.

### Best Mode for Carrying Out the Invention

Some of the best modes for carrying out this invention will be described, with reference to the accompanying drawings.

### [Fist Embodiment]

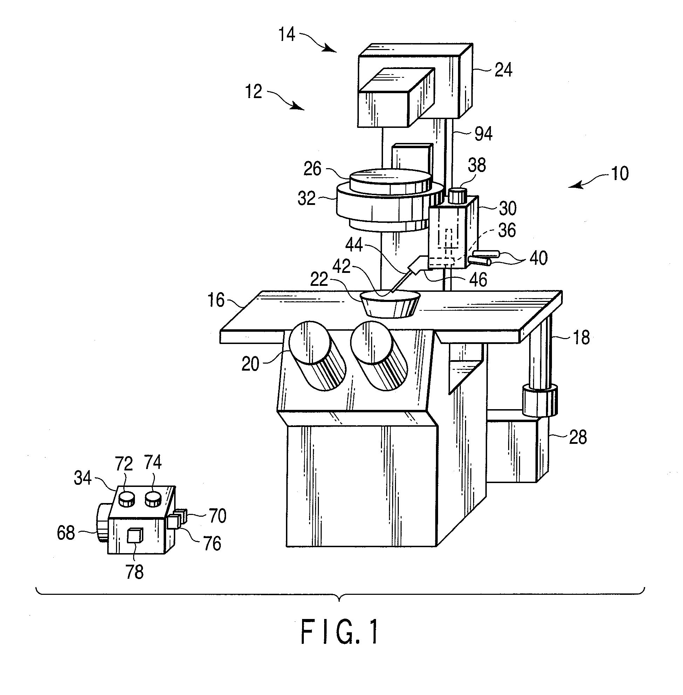

As shown in FIG. 1, a tip drive apparatus 10 according to a first embodiment of this invention is attached to an inverted microscope 12 through which to observe cells. So attached, the tip drive apparatus 10 is used.

The inverted microscope 12 has an illumination device 14, a microscope XY stage 16, a microscope XY stage handle 18, an objective lens (not shown), and an eyepiece 20. The illumination device 14 illuminates the cells set in on a dish 22. The microscope XY stage 16 moves the dish 22 in X direction and Y direction. When operated, the microscope XY stage handle 18 drives the microscope XY stage 16. The objective lens and the eyepiece 20 constitute an optical system through which to observe the light reflected from, or passing through, the cells mounted on the dish 22, or the fluorescent light emanating from the cells. At least the bottom of the dish 22 is made of transparent material such as glass, so that the cells may be observed.

The inverted microscope 12, which is a manually operable type, may be replaced by an electrically-driven type, in which the XY stage 16 is driven and controlled by a computer. Further, the inverted microscope 12 may instead be a type that has a CCD camera and can display images on a monitor.

The illumination device 14 has an illumination light source 24, a condenser lens 26, and an epi-illumination light source 28. The illumination light source 24 applies illumination light to the cells mounted on the dish 22, from the side opposite to the eyepiece 20. The condenser lens 26 receives the illumination light emitted from the illumination light source 24 and converges the light onto the cells. The epi-illumination light source 28 applies illumination light to the cells mounted on the dish 22, from the same side as the eyepiece 20.

The tip drive apparatus 10 according to this embodiment is composed of a main unit 30, a microscope adaptor 32, and an operation module 34. The microscope adaptor 32 is a unit that is attached to the condenser lens 26 of the main unit 30. As seen from FIG. 1, the main unit 30 is attached at the right side of the condenser lens 26, in front of the inverted microscope 12, where the eyepiece 20 is arranged. The operation module 34 is connected by a cable (not shown) to the main unit 30 and can be set at any desired position.

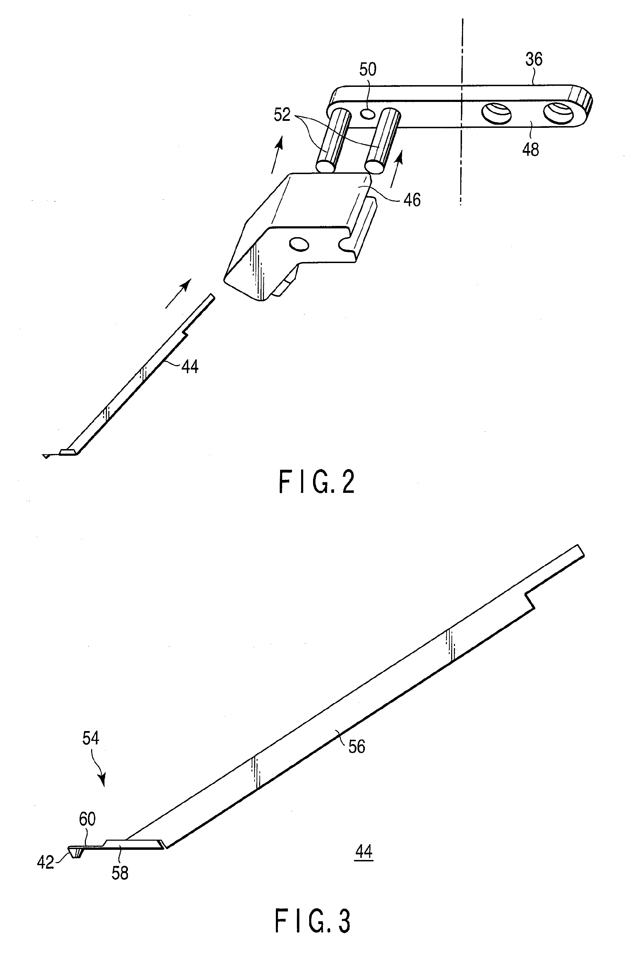

The main unit 30 has an adaptor holder unit 36, a Z-drive unit 38, and a needle-tip XY adjustment knob 40. A tip unit 42, which should be driven, is secured to a needle 44. The needle 44, which has the tip unit 42, is attached to the adaptor 46. The adaptor 46 holding the needle 44 is attached to the adaptor holder unit 36. As the adaptor holder unit 36 is moved in the Z-direction, the Z-drive unit 38 drives the tip unit 42 in the Z-direction. The needle-tip XY adjustment knob 40 moves the adaptor holder unit 36 in X direction and Y direction, adjusting the XY position of the tip unit 42.
As shown in FIG. 2, the adaptor holder unit 36 has a Z-axis drive holder unit 48 configured to secure it to a linear movement mechanism (not shown) of the Z-drive unit 38 through a XY drive mechanism (not shown) (the needle-tip XY adjustment knob 40 drives the adaptor holder unit 36, in cooperation with the drive mechanism). Moreover, the adaptor holder unit 36 has an attachment member on the side opposite to the Z-axis drive holder unit 48 in the lengthwise direction. The attachment member is configured to attach the adaptor 46 to, and detached the same from, the adaptor holder unit 36. The attachment member is a magnet 50 if the adaptor 46 is made of metal or has a metal component. That section of the adaptor holder unit 36, which is illustrated on the right of the one-dot, dashed line shown in FIG. 2, is incorporated in the main unit 30. That is, the magnet 50 is provided outside the main unit 30. Near the magnet 50, fitting members 52 are provided, and can fit into the holes or grooves made in the adaptor 46 to set the adaptor 46 at a desired position. The fitting members 52 protrude toward the front of the inverted microscope 12. Therefore, the adaptor 46 is attached to the adaptor holder unit 36 by inserting into the holes or grooves from the front of the inverted microscope 12.

Another magnet 50 and other fitting members 52 may be provided on the back of the adaptor holder unit 36 so that the adaptor 46 may be attached to the adaptor holder unit 36 when the main unit 30 is secured to the left side of the condenser lens 26. Alternatively, the adaptor holder unit 36 may be replaced by another, depending upon the position at which the main unit 30 is secured.

As shown in FIG. 3, the needle 44 attached to the adaptor 46 is composed of a cantilever tip 54 and a shaft 56 holding the cantilever tip 54. The cantilever tip 54 has the tip unit 42 mentioned above. The cantilever tip 54 is adhered to the distal end of the shaft 56.

The cantilever tip 54 has been manufactured by a silicon process and is composed of a silicon base unit 58, a flexible lever unit 60, and the above-mentioned tip unit 42. The silicon base unit 58 is a part to which another part, i.e., the shaft 56 is adhered. The lever unit 60 extends from the silicon base unit 58 and has, for example, thickness of 2.7 µm, length of 240 µm and elastic constant of about 2 N/m. The tip unit 42 is formed at the free end of the lever unit 60, at an angle of about 90° to the lengthwise direction of the lever unit 60.

In the tip drive apparatus 10 according to this embodiment, the needle 44 is inserted into, and held in, the hole (not shown) made in the adaptor 46. Thereafter, the adaptor 46, now holding the needle 44, is attached to the main unit 30. The needle 44, which is basically an expendable article and replaced frequently, can thus be replaced by a new one. Therefore, the tip drive apparatus 10 can be used over again, without the risk of contamination.

Assume that the needle 44, which is a thin and long member, is directly attached to the main unit 30. Then, the operability decreases, and the tip unit 42 may hit a part, such as microscope XY stage 16, of the inverted microscope 12 during the attaching operation, and may possibly be broken while being attached to the main unit 30. In this embodiment, the needle 44 is first attached to the adaptor 46 removed from the main unit 30, and the adaptor 46 is then secured at the front of the main unit 30. Hence, the risk of damaging the tip unit 42 can be reduced.

The adaptor 46 is configured to hold the shaft 56 of the needle 44, at a prescribed angle and in a downwardly inclined position, when the adapter is attached to the main unit 30. Further, the cantilever tip 54 is adhered to the shaft 56, at a prescribed angle to the shaft 56. Moreover, as pointed out above, the tip unit 42 is provided, extending in a direction to intersecting with the lengthwise direction of the lever unit 60. The tip unit 42 is therefore held with its distal end directed downward, almost in the vertical direction, at the free end of the lever unit 60, as long as the adaptor 46 remains secured to the main unit 30.

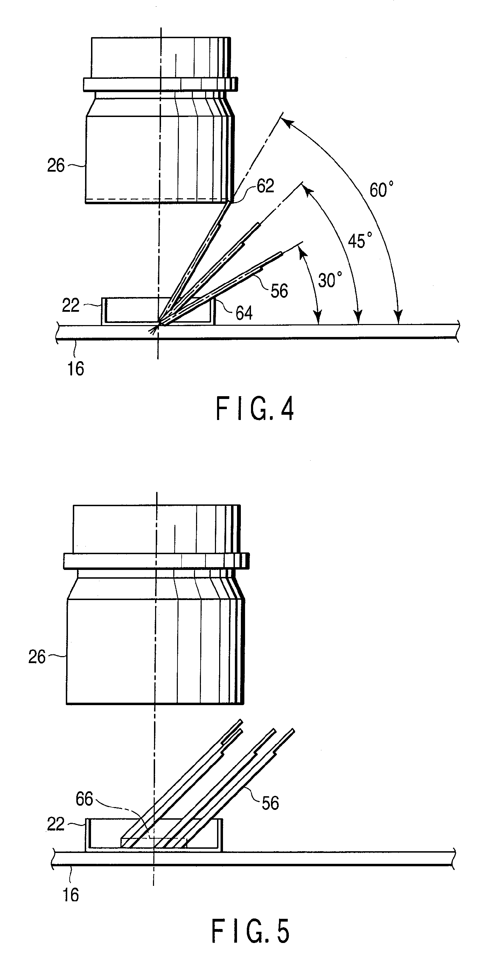

The angle at which the adaptor 46 holds the shaft 56 is determined as will be explained below. If the shaft angle indicated in FIG. 4 is too large, it will inevitably interfere with the condenser lens 26. Assume that the needle 44 is, for example, about 50 mm long. Then, the shaft 56 interferes with the condenser lens 26 if the shaft angle larger is larger than 60°. Conversely, if the shaft angle larger is too small, the shaft 56 inevitably interferes with the sidewall of the dish 22 as indicated by reference number 64 in FIG. 4. The dish 22 may be a glass-bottom of 35 mm dish that is usually used in cell culture. In this case, the shaft 56 interferes with the dish 22 if it is rotated upward by less than 30°. This is why the adaptor 46 holds the shaft 56 at the angle of 45° that is the intermediate value between 30° and 60°.

If the adaptor 46 holds the shaft 56 at the angle of 45°, there will be provided such a movement region 66 as indicated by a one-dot, dashed line shown in FIG. 5. The work can be proceeded, without the risk that the glass surface (having a diameter of about 14 mm) of the glass bottom dish of 35 mm interferes with the condenser lens 26 or the sidewall of the dish 22.

Thus, the angle at which the adaptor 46 holds the shaft 56 is determined to enable the needle 44 to have an sufficient movement region 46, in consideration of possible interference with the condenser lens 26 and the dish 22 used. The adaptor 46 has a hole (not shown) so shaped that the needles 44 may be inserted and held the shaft 56 at the angle so determined.

As shown in FIG. 1, the operation module 34 of the tip drive apparatus 10 has a Z-value adjustment handle 68, a speed setting dial 70, a minute-adjustment (up) button 72, a minute-adjustment (down) button 74, a movement setting dial 76, and a Z-value setting button 78.

The Z-value adjustment handle 68 and speed setting dial 70 are used to move coarsely the adaptor holder unit 36 in the Z-direction (in units of millimeters). As the Z-value adjustment handle 68 is rotated, the Z-drive unit 38 drives the adaptor holder unit 36 in the Z-direction, in accordance with the rotation of the Z-value adjustment handle 68. When operated, the speed setting dial 70 switches the distance by which to move the unit 36 as the Z-value adjustment handle 68 is rotated, from any one of the three values, i.e., long, intermediate and short, to another value.

The minute-adjustment buttons 72 and 74 and movement setting dial 76 are used to move minutely the adaptor holder unit 36 in the Z-direction (in units of microns). As the minute-adjustment (up) button 72 or minute-adjustment (down) button 74 is operated, the Z-drive unit 38 drives the adaptor holder unit 36 minutely in the Z-direction, in accordance with the operation of the button. The movement setting dial 76 switches the distance by which to move the unit 36 as the minute-adjustment button 72 or 74 is operated one time, from any one of the three values, i.e., long, intermediate and short, to another value.

The Z-value setting button 78 is a button, which may be pushed to instruct that any position in the Z-direction be stored. Even if the Z-value adjustment handle 68 or the minute-adjustment button 72 or 74 is operated, the adaptor holder unit 36 will never move down below the position stored by pushing the Z-value setting button 78 (toward the sample placed in the dish 22). The Z-value setting button 78 has a latch mechanism (not shown). Once depressed or turned on by the operator, the Z-value setting button 78 remains in the on state until it is depressed again. Hereinafter, the operation of the Z-value adjustment handle 68 and the minute-adjustment buttons 72 and 74 while the Z-value setting button 78 remains in the off state is called the "first mode," and the operation of the Z-value adjustment handle 68 and the minute-adjustment buttons 72 and 74 while the Z-value setting button 78 remains in the on state is called the "second mode."

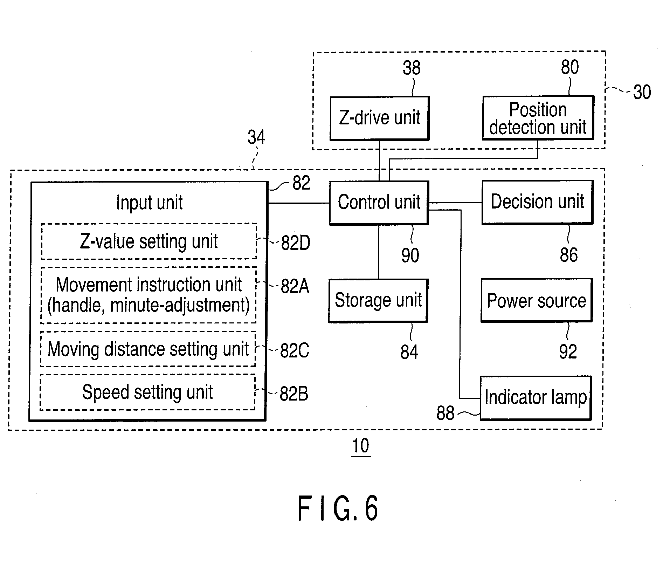

As shown in FIG. 6 illustrating the electrical configuration of the tip drive apparatus 10 according to this embodiment, the main unit 30 has, in addition to the Z-drive unit 38, a position detection unit 80 that is configured to detect the position of the adaptor holder unit 36. The position detection unit 80 may directly detect the position of the adaptor holder unit 36 by using optical means, or may indirectly detect the position of the adaptor holder unit 36 by detecting the distance by which the Z-drive unit 38 has been driven. Furthermore, the position detection unit 80 may be provided as a unit separated from the main unit 30.

The operation module 34 has an input unit 82, a storage unit 84, a decision unit 86, an indicator lamp 88, a control unit 90, and a power source 92.

The input unit 82 includes a movement instruction unit 82A, a speed setting unit 82B, a moving distance setting unit 82C, and a Z-value setting unit 82D. The movement instruction unit 82A outputs a movement instruction when the Z-value adjustment handle 68 is operated and the minute-adjustment button 72 or 74 is turned on. The speed setting unit 82B outputs a speed setting signal representing the moving speed set as the speed setting dial 70 is rotated. The moving distance setting unit 82C outputs a distance setting signal representing the distance set as the movement setting dial 76 is rotated. The Z-value setting unit 82D outputs a Z-value setting signal when the Z-value setting button 78 is turned on. The signals output from the input unit 82 are input to the control unit 90.

The storage unit 84 stores, as the Z-value, the position of the adaptor holder unit 36, which the position detection unit 80 detects when the Z-value setting button 78 is turned on. The decision unit 86 compares the position of the adaptor holder unit 36, which the position detection unit 80 has detected, with the Z-value stored in the storage unit 84, thereby determining whether the adaptor holder unit 36 has reached the position of the Z-value. The indicator lamp 88 blinks in response to the Z-value setting signal output from the Z-value setting unit 82D. Seeing the indicator lamp 88 blinking, the operator can confirm that the Z-value has been duly stored.

The control unit 90 controls the entire tip drive apparatus 10. The power source 92 supplies power to the components of the tip drive apparatus 10.

A tip driving method, which uses the tip drive apparatus 10 according to this embodiment, will be explained below.

Here, a case will be described, in which the tip drive apparatus 10 according to this embodiment is used to introduce a substance into cells being cultured in a culture solution filled in the dish 22.

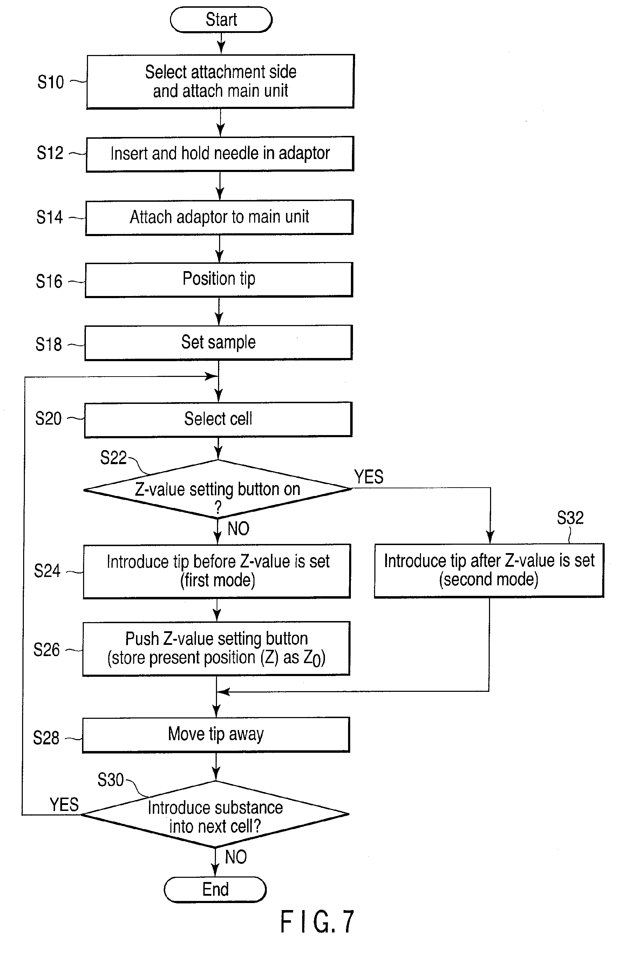

As shown in FIG. 7, the side to which the main unit 30 should be attached is selected, and the main unit 30 is then attached to the condenser lens 26 via the microscope adaptor 32 (Step S10).

Next, the needle 44 is inserted into, and held in, the adaptor 46 removed from the main unit 30 (Step S12). The adaptor 46 holding the needle 44 is attached to the adaptor holder unit 36 of the main unit 30, from the front of the inverted microscope 12 (Step S14).

Thereafter, the tip is positioned (Step S16). That is, while observing the needle 44, the operator brings the tip unit 42 formed at the distal end of the needle, to the center (i.e., view-field center) of the eyepiece (not shown). The operator accomplishes this by manipulating the needle-tip XY adjustment knob 40 of the main unit 30 and the Z-value adjustment handle 68 of the operation module 34. This manipulation is performed, not having the dish 22 mounted on the microscope XY stage 16. As for the Z-direction, the operator turns the speed setting dial 70 of the operation module 34, setting the long or intermediate distance, and then operates the Z-value adjustment handle 68, thereby lowering the tip unit 42 to a position where he or she can see the lever unit 60 of the cantilever tip 54.

When the tip unit 42 is so positioned, a sample is set, more precisely, the dish 22 is mounted on the microscope XY stage 16 (Step S18). This step is performed in the following sequence. First, the Z-value adjustment handle 68 of the operation module 34 is operated, moving the tip unit 42 at the distal end of the needle 44, to a safe region (upward in the Z-direction). An arm 94 (FIG. 1) of the inverted microscope 12 is then pulled back. As a result, the main unit 30 is moved as a whole. A space for sample setting is thereby provided. Then, the dish 22 (sample) is mounted on the microscope XY stage 16. Thereafter, the arm 94 of the inverted microscope 12 is moved to the initial position. Note that the dish 22 (sample) so set contains a substance in a dispersed state, which will be introduced into cells being cultured in a culture solution held in the dish 22.

Next, the cell into which the substance should be introduced is selected (Step S20). First, the operator manipulates the microscope XY stage handle 18, moving the microscope XY stage 16 and, thereby, arranging the cell held in the dish 22 into the view field of the microscope so that the cell may be observed. Thereafter, the operator actuates the Z-drive unit 38, moving the tip unit 42 of the needle 44 toward the cell from above. That is, while observing through the eyepiece 20, the operator lowers the tip unit 42 in the Z-direction until the lever unit 60 of the cantilever tip 54 comes into the view field and become visually confirmed. This is achieved by first turning the speed setting dial 70 of the operation module 34, setting the low speed, and then operating the Z-value adjustment handle 68. Since the cells in the dish 22 are not at the same height as the tip unit 42, the tip unit 42 is not focused and can hardly be observed. Therefore, the operator moves the tip unit 42 down in the Z-direction, using the lever unit 60 as index. This is because the lever unit 60 is larger than the tip unit 42 and therefore the lever unit 60 can be recognized generally, even if its image is not focused. After the lever unit 60 moves to a position where it is visually recognized, the operator adjust the position of the microscope XY stage 16 with respect to X direction and Y direction, while observing the cells through the eyepiece 20. The tip unit 42 is thereby set at a position that seems right above the cell into which to introduce the substance. Thus, the cell into which to introduce the substance is selected (determined).

The following operation depends on whether the Z-value has been set or not in the storage unit 84 of the operation module 34.

When the tip is driven for the first time, the Z-value has yet to be set in the storage unit 84 (Step S22). Therefore, the tip is introduced in the first mode (without using the Z-value) (Step S24). That is, the operator determines an optimal position in the Z-direction, while operating the Z-value adjustment handle 68 or the minute-adjustment button 72 or 74 and observing through the eyepiece 20, confirming "the distortion of the cell" or "the bending of the lever unit 60." At this point, the Z-value adjustment handle 68 is manipulated, while the speed setting dial 70 is turned, switching the speed from any one of the three values, i.e., high, medium and low, to another value. The minute-adjustment button 72 or button 74 is operated, while the movement setting dial 76 is turned, switching the distance from any one of the three values, i.e., long, intermediate and short, to another value.

As the tip unit 42 is thus moved down toward the bottom of the dish 22, lowering the distal end of the tip unit 42, the tip unit 42 contacts the cell in the dish 22. If the tip unit 42 is further lowered, the distal end of the tip unit 42 will pass through the cell membrane and penetrates the cell nucleus, forming a scar or hole in the membrane and nucleus. The substance dispersed in the dish 22 therefore flows into the cell through the formed scar or hole. The substance may flow into the cell, without forming a scar or hole, depending on the size of particles to introduce, if the channel coupled to a stretch receptor or the like is opened when the tip unit 42 deforms the cell, applying a physical stimulus to the cell. Thus, the substance is introduced.

When the substance is so introduced, the operator may push the Z-value setting button 78 of the operation module 34. Then, the control unit 90 of the operation module 34 determines that the Z-value setting button 78 has been pushed. In this case, the control unit 90 makes the storage unit 84 stores, as the Z-value representing an optimal position, the present position of the adaptor holder unit 36, which the position detection unit 80 has detected (Step S26). At this point, the control unit 90 turns on the indicator lamp 88.

Thereafter, the operator operates the Z-value adjustment handle 68 of the operation module 34, raising the needle 44 and, thus, moving up the tip unit 42 (Step S28). That is, the operator turns the speed setting dial 70 of the operation module 34, switching the speed to the medium speed or the low speed, and then operates the Z-value adjustment handle 68, raising the tip unit 42.

After the tip unit 42 is raised and pulled from the cell, and after a certain time has passed, the cell membrane is restored by itself and now contains the substance.

Assume that the tip unit 42 has been completely moved up (Step S28). Then, whether the substance should be introduced into the next sample cell is determined (Step S30). If NO, the operator turns off the power switch (not shown) of the main unit 30, terminating the tip driving method.

On the other hand, if the substance should be introduced into any other cell (Step S30), the method returns to Step S20. Thus, the substance may be introduced into other sample cells, one after another. That is, the operator manipulates the microscope XY stage handle 18, while making observation through the eyepiece 20, thereby actuating the microscope XY stage 16 and setting the tip unit 42 right above the cell into which to introduce the substance. In other words, the operator selects the cell into which the substance should be introduced (Step S20).

At the time the tip is driven for the second time, and so forth, the Z-value has already been set in the storage unit 84 (Step S22). Therefore, the tip is introduced in the second mode (by using the Z-value) (Step S32). In this case, the Z-value has been set in the storage unit 84. Therefore, the operator can lower the tip unit 42 to an optimal position, merely by fully lowering the tip unit 42, without worrying about an excessive manipulation of the Z-value adjustment handle 68 and minute-adjustment buttons 72 and 74, once the tip unit 42 has been positioned in the horizontal direction. That is, the decision unit 86 of the operation module 34 compares the position of the adaptor holder unit 36, detected by the position detection unit 80, with the Z-value set in the storage unit 84, determining whether the adaptor holder unit 36 (tip unit 42) has reached the position of the Z-value. If the decision unit 86 determines that the adaptor holder unit 36 is found to have reached this position, the control unit 90 of the operation module 34 controls the Z-drive unit 38, preventing the same from moving down further, even if the Z-value adjustment handle 68 and the minute-adjustment buttons 72 and 74 are operated.

Since the optimal Z-position is set in the storage unit 84, the adaptor holder unit 36 (tip unit 42) may be automatically lowered to the optimal Z-position. That is, the handle operation in the second mode may be automated.

If the inverted microscope 12 is an electrically-driven type, a computer controls and drives the microscope XY stage 16. In this case, the inverted microscope 12 has a CCD camera or the like, and can display images on a monitor. If the inverted microscope 12 is such an electrically-driven type, not a manually operable type, any cell into which the substance should be introduced may be selected on the monitor screen, and the tip unit may be automatically moved to its position. In other words, the microscope XY stage 16 may be automatically adjusted in the XY direction.

Note that the substance to introduce into the cell may be anything that can be dispersed in the dish 22, such as genes, dyes, fluorescent reagent, e.g., quantum dots, ions, peptides, proteins or polysaccharides.

### [Examples]

A first example is HelaS3 cells which were immersed in a gene solution, and into which genes were introduced. The genes express GFP fluorescent protein. Whether the genes were successfully introduced or not can be confirmed by performing fluorescence observation.

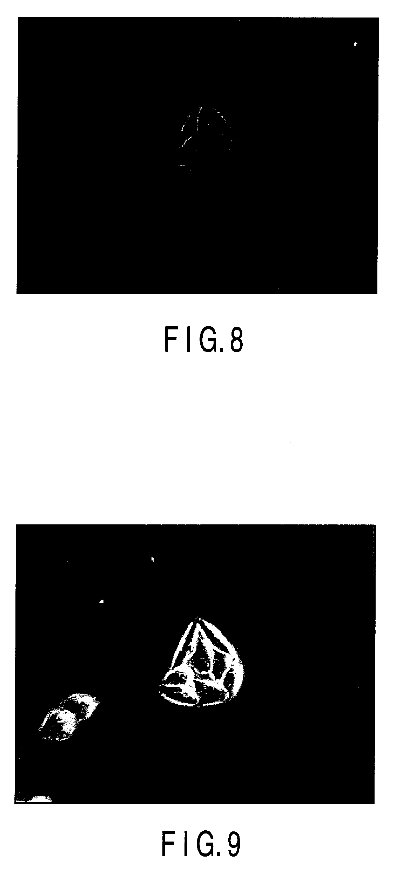

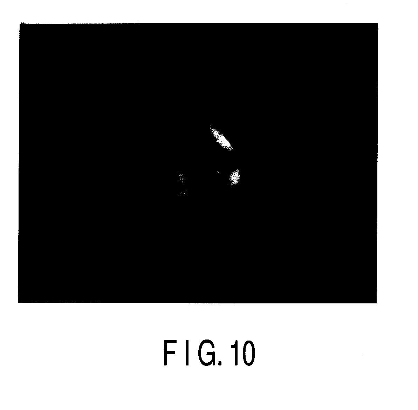

FIG. 8 presents a microscope image of the cells, obtained immediately after genes had been introduced. FIG. 9 and FIG. 10 are microscope images of the cells, acquired 24 hours after introducing the genes, which show whether the genes have been introduced into the cells. The image of FIG. 9 is one observed through a phase contrast microscope, showing the state the cells had 24 hours after the genes had been introduced.
FIG. 10 is one obtained through fluorescence observation. In the cell into which the genes had been successfully introduced, the genes well expressed, attaining intense fluorescent light. This proves that the genes were introduced into the cells at a very high efficiency.

Hence, the tip drive apparatus 10 according to this embodiment can introduce a substance into a cell, not only in such a low-invasive manner and maintaining the cell at such a high survival rate as the conventional method, but also with high reliability and high efficiency.

Moreover, the needle 44 can be replaced by a new one in the tip drive apparatus 10 according to this embodiment. The tip drive apparatus 10 can therefore be repeatedly used, without the risk of contamination.

Further, the needle 44 has a long and thin shaft 56 in the tip drive apparatus 10 according to this embodiment. This helps to minimize the intrusion into the illumination region, due to the condenser lens 26.

Still further, in the tip drive apparatus 10 according to this embodiment, the needle 44 is attached to the adaptor 46 which has been removed from the main unit 30, and then the adapter 46 holding the needle 44 is secured at the front of the main unit 30. The frontal approach of the adaptor 46, accomplished by using the fitting members 52, solves the problem that the operability decreases due to the use of the shaft 56 that has a long reach. This can reduce the risk that the tip unit 42 may be broken while the needle 44 is being attached to the adaptor 46.

Furthermore, the adaptor 46 holds the needle 44 at a prescribed angle in the tip drive apparatus 10 according to this embodiment. This provides a large region 66 in which the needle 44 can move, without interfering with the condenser lens 26 and the dish 22 used.

### [Second Embodiment]

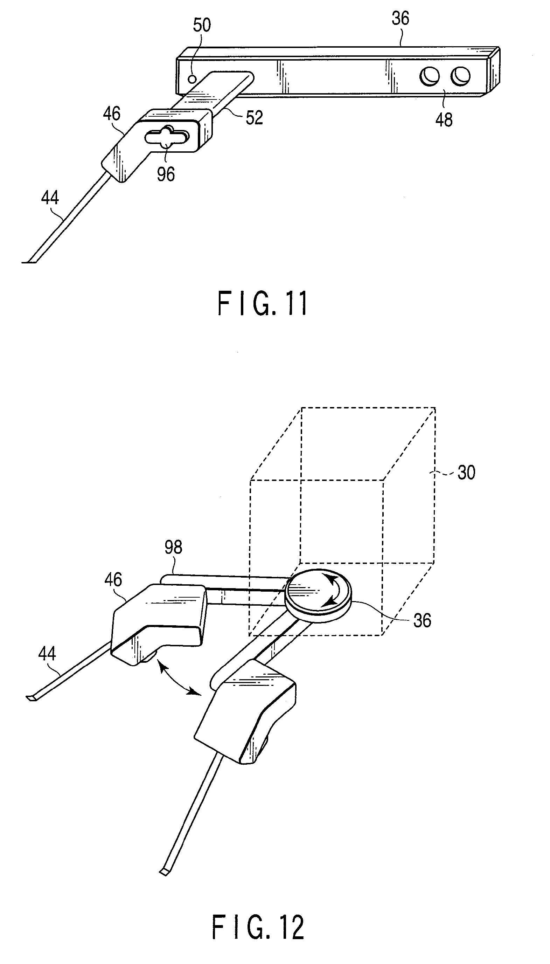

In a tip drive apparatus according to a second embodiment of this invention, the adaptor 46 is not detached from the main unit 30 as in the first embodiment, but can move forward in parallel, while being fitted on fitting members 52 as shown in FIG. 11. In this case, the fitting member 52 extends forward, longer than in the first embodiment, and has a stopper 96 at the distal end, the stopper being configured to prevent the adaptor 46 from slipping off. The fitting members 52 have an elliptical cross section. This prevents the adaptor 46 from rotating to change the angle at which the adaptor 46 holds the needle 44.

In the tip drive apparatus according to this embodiment, to attach the needle 44 to the adaptor 46, the adaptor 46 is pulled forward and is removed right below the condenser lens 26. Then, the needle 44 is inserted into the hole (not shown) made in the adaptor 46 and held in the hole. Thereafter, the adaptor 46 is moved in horizontal direction toward the adaptor holder unit 36 and finally secured to the magnet 50.

The tip drive apparatus according to this embodiment can introduce a substance into a cell, not only in such a low-invasive manner and maintaining the cell at such a high survival rate as the conventional method, but also with high reliability and high efficiency.

Since the needle 44 can be replaced by a new one, the tip drive apparatus can therefore be repeatedly used, without the risk of contamination.

Further, the needle 44 has a long and thin shaft. This helps to minimize the intrusion of eth condenser lens 26 into the illumination region.

Since the adaptor 46 can move in parallel, it can be pulled from the main unit 30, and the needle 44 can then be attached to the adaptor 46. Therefore, the frontal approach of the adaptor 46, accomplished by using the fitting member 52, solves the problem that the operability decreases due to the use of the shaft 56 that has a long reach. This can reduce the risk that the tip unit 42 may be broken while the needle 44 is being attached to the adaptor 46.

Still further, since the adaptor 46 holds the needle 44 at a prescribed angle, a large region 66 can be provided, in which the needle 44 can move without interfering with the condenser lens 26 and the dish 22 used.

Furthermore, the adaptor 46 can hold the needle 44 at a prescribed angle. This is because the apparatus has a horizontal position setting mechanism, such as magnet 50, which holds the adaptor 46 at a prescribed position, and an angle setting mechanism, such as fitting member 52, which has such a cross section as prevents the adaptor 46 from rotating.

The horizontal position setting mechanism is not limited to the magnet 50. The angle setting mechanism is not limited to the adaptor 46 having an elliptical cross section. These mechanisms can, of course, be provided in any other configurations.

### [Third Embodiment]

In a tip drive apparatus according to a third embodiment of this invention, the adaptor 46 is secured to a rotating arm 98 as shown in FIG. 12, not being one that can be removed from the main unit 30 as in the first embodiment. The root of the rotating arm 98 is attached to the adaptor holder unit 36, which secured to a linear movement mechanism (not shown) of the Z-drive unit 38, at the lower surface of the main unit 30, and which can rotate in the direction of the arrow. The adaptor holder unit 36 is driven by the Z-drive unit 38, downward from the lower surface of the main unit 30.

In the tip drive apparatus according to this embodiment, to attach the needle 44, the Z-drive unit 38 is driven, raising the adaptor holder unit 36 to the lower surface of the main unit 30. Thereafter, the adaptor 46 is rotated forward. In this state, the needle 44 is inserted into, and held in, the hole (not shown) made in the adaptor 46. Then, the adaptor 46 is rotated in the direction of the arm 94 of the inverted microscope 12. The tip unit 42 at the distal end of the needle 44 is thereby arranged right below the condenser lens 26 (that is, in the view field).

It is desirable to provide a position setting mechanism (not shown) that holds the rotating arm 98 so that the tip unit 42 may lie in the view field. The rotating arm 98, for example, may be made of metal. In this case, the position setting mechanism can be a magnet attached to the lower surface of the main unit 30.

The tip drive apparatus according to the present embodiment can introduce a substance into a cell, not only in such a low-invasive manner and maintaining the cell at such a high survival rate as the conventional method, but also with high reliability and high efficiency.

Since the needle 44 can be replaced by a new one, the tip drive apparatus 10 can therefore be repeatedly used, without the risk of contamination.

Further, the needle 44 has a long and thin shaft. This helps to minimize the intrusion of eth condenser lens 26 into the illumination region.

Since the adaptor 46 is rotated from the view field, and the needle 44 is then attached to the adaptor 46. Therefore, the frontal approach of the adaptor 46 solves the problem that the operability decreases due to the use of the shaft 56 that has a long reach. This can reduce the risk that the tip unit 42 may be broken while the needle 44 is being attached to the adaptor 46.

Furthermore, since the adaptor 46 holds the needle 44 at a prescribed angle, a large region 66 can be provided, in which the needle 44 can move without interfering with the condenser lens 26 and the dish 22 used.

In addition, the adaptor 46 can reliably hold the needle 44 at the prescribed angle, because the position setting mechanism holds the rotating arm 98 to position the tip unit 42 in the view field and also because the adaptor 46 is secured to the rotating arm 98.

The adaptor 46 may be removably coupled to the rotating arm 98 as in the first embodiment. If this is the case, a position setting mechanism may be provided, which is composed of a magnet 50 and a fitting member 52 as in the first embodiment.

### [Fourth Embodiment]

In the first to third embodiments described above, the adaptor 46 holds the needle 44 at a prescribed angle to the dish 22 (sample). The needle 44 need not always held at that angle, nonetheless. The needle 44 only needs to be held at that angle, only while the tip is being driven.

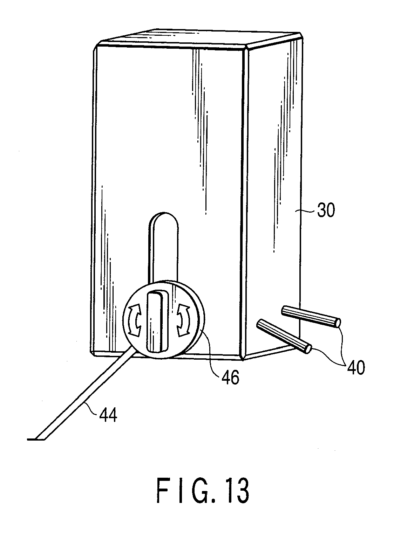

Therefore, the adaptor 46 is a dial-type as shown in FIG. 13 in a tip drive apparatus according to a fourth embodiment of this invention. The adaptor 46 can hold the needle at any desired angle. In this case, the adaptor 46 may be detachable from the main unit 30 or may not be detachable from the main unit 30.

If the adaptor 46 cannot be detached from the main unit 30, the needle 44 may be attached in the following manner. First, the dial-type adaptor 46 is rotated, changing the angle of the hole (not sown) for holding the needle 44 and moving the hole out of the view field of the microscope. The needle 44 is then inserted into the hole, for example, sideways. Thereafter, the adaptor 46 rotated, setting the needle 44 to the tip-driving angle. Then, the tip unit 42 at the distal end of the needle 44 is arranged right below the condenser lens 26 (in the view field).

If the adaptor 46 is a detachable one, the needle 44 may be attached to the adapter 46 after the adaptor 46 has been removed as explained in conjunction with the first embodiment. In this case, it is desired that the adaptor 46 holding the needle 44 should be attached to the main unit 30 in the following sequence, in order to prevent the tip unit 42 from contacting the inverted microscope 12 and from being broken. That is, the adaptor 46 is first attached to the main unit 30 at such an angle that the needle 44 may extend in horizontal direction. Next, the adaptor 46 is rotated and held so that the tip may be driven. Then, the tip unit 42 at the distal end of the needle 44 is arranged right below the condenser lens 26 (in the view field).

The tip drive apparatus according to the fourth embodiment can introduce a substance into a cell, not only in such a low-invasive manner and maintaining the cell at such a high survival rate as the conventional method, but also with high reliability and high efficiency.

Since the needle 44 can be replaced by a new one, the tip drive apparatus 10 can therefore be repeatedly used, without the risk of contamination.

Further, the needle 44 has a long and thin shaft. This helps to minimize the intrusion of eth condenser lens 26 into the illumination region.

Since the adaptor 46 is rotated, and the needle 44 is then attached to the adaptor 46. Therefore, the approach of the adaptor 46 at a changed angle solves the problem that the operability decreases due to the use of the shaft 56 that has a long reach. This can reduce the risk that the tip unit 42 may be broken while the needle 44 is being attached to the adaptor 46.

Moreover, the adaptor 46 can reliably hold the needle 44 at a prescribed angle, without causing the needle 44 to interfere with the dish 22, because the adaptor 46 holds the needle 44 at the prescribed angle while the tip is being driven.

Further, the adaptor 46 can reliably hold the needle 44 at the prescribed angle, because the dial-type adaptor 46 is set at a position within the view field.

### [Fifth Embodiment]

In the first to fourth embodiments described above, one tip drive apparatus 10 is secured to the inverted microscope 12 and is used. A plurality of tip drive apparatuses 10 may be used at the same time. For example, main units 30 may be secured to the sides of the condenser lens 26.

If a plurality of tip drive apparatuses 10 are used in this manner, tips can be used not only to introduce substances, but also to, for example, apply a potential difference between the tip units 42, thereby to give cells an electrical stimulus. The electrical stimulus is not necessarily be given exclusively by using a plurality of tip units 42. Rather, it can be given by applying a potential difference between a tip unit 42 and a particular electrode (e.g., glass bottom with ITO). If this is the case, the tip unit 42 had better be electrically conductive.

Thus, this embodiment can apply an electrical stimulus to cells, not only in such a low-invasive manner and maintaining the cell at a high survival rate, enabling the operator to observe the living cells with high efficiency.

This invention has been explained, with reference to various embodiments. The invention is not limited to the embodiments described above, nevertheless. Various changes and modifications can, of course, be made within the scope of this invention.

For example, the main unit 30 of the tip drive apparatuses 10 may be attached to a support unit supporting the condenser lens 26, not to the microscope adaptor 32 secured to the condenser lens 26 as in the embodiments described above.

## Claims

1. A tip drive apparatus (10) capable to moving a tip unit (42) toward an object, while holding the tip unit at a prescribed angle, the tip unit being formed on a support unit (60) having flexibility and directed to the object at the prescribed angle, the apparatus comprising:
a needle (44) having a shaft (56) to which the support unit on which the tip unit is formed; and
an adaptor (46) for holding the needle, enabling the needle to be replaced by another,
**characterized in that** the adaptor is configured to be able to change a positional relation with respect to the main unit (30) of the tip drive apparatus, while the needle is being attached.

2. The tip drive apparatus according to claim 1, **characterized in that** the adaptor is detachably coupled to the main unit of the tip drive apparatus, and the change of the positional relation of the adaptor includes attachment of the adaptor to, and detachment it from, the main unit of the tip drive apparatus.

3. The tip drive apparatus according to claim 2, **characterized by** further comprising a fastening mechanism (50) for fastening the adaptor to the main unit of the tip drive apparatus, thereby to set the tip unit in a prescribed positional relation and to hold the tip unit at the prescribed angle.

4. The tip drive apparatus according to claim 1, **characterized in that** the change of the positional relation of the adaptor includes movement of the adaptor to set the needle in a region where the needle is easily attached to the adaptor.

5. The tip drive apparatus according to claim 4, **characterized in that** the movement of the adaptor includes rotation of the adaptor.

6. The tip drive apparatus according to claim 5, **characterized by** further comprising a fastening mechanism (50) for fastening the adaptor to the main unit of the tip drive apparatus, at a prescribed rotation angle with respect the main unit.

7. The tip drive apparatus according to claim 4, **characterized in that** the movement of the adaptor includes horizontal movement of the adaptor.

8. The tip drive apparatus according to claim 7, **characterized by** further comprising a fastening mechanism (50) for fastening the adaptor to the main unit of the tip drive apparatus, at a prescribed position with respect the main unit.
